# EUROPEAN PATENT APPLICATION

(11) **EP 4 715 680 A2**
(43) Date of publication of application: **25.03.2026**
(21) Application number: 25198718.6
(22) Date of filing: 28.08.2025
(51) Int. Cl.: G06N 3/123, G16B 15/10

(54) **THERMODYNAMICALLY FAVOURED MOLECULAR COMPUTATIONS**

(30) Priority: 28.08.2024 GB 202412584
(71) Applicant: National University of Ireland, Maynooth, Maynooth, County Kildare (IE)
(72) Inventor: Woods, Damien, Maynooth (IE); Stérin, Tristan, Maynooth (IE); Eshra, Abeer, Maynooth (IE)
(74) Representative: Barker Brettell LLP

(57) **Abstract**

A method 800 for making molecular computations comprises: i) providing a mixture 100 comprising a scaffold 110 comprising N scaffold positions 120; ii) designing a set of computing tiles to drive a desired computation, the set of computing tiles comprising at least N different computing tile types, wherein the computing tile types are selected to be used for the computation, in such a way that a target output has a higher probability of being reached than the probability for any other potential output, wherein for each computing tile 140, a bottom position domain 160 is arranged to bind directly to a matching scaffold position 120 with a first binding strength, and to the compute domains 150 of other computing tiles 140 with a set of second binding strengths, which are each weaker than the first binding strength; iii) adding the designed set of computing tiles to the mixture 100; iv) allowing computing tiles 140 to bind to scaffold positions 120 until all scaffold positions 120 required for the computation have been filled; v) allowing replacement of all mismatched computing tiles 140, based on correct compute domain bindings being enthalpically favoured over incorrect compute domain bindings; and vi) reaching an output configuration.

## Description

### TECHNICAL FIELD

The present disclosure relates generally to methods and arrangements for making molecular computations, especially DNA computations.

### BACKGROUND

DNA is an incredibly dense storage medium for digital data. Each gram of DNA could store up to exabytes of data, which could stay readable for thousands of years. DNA origami uses molecular building blocks for engineering self-assembling materials using nucleic acid nanotechnology. Watson-Crick base pairing, with guanine (G) forming a base pair with cytosine (C), and adenine (A) forming a base pair with thymine (T), allows a combinatorically large set of nucleotide sequences to be used when designing binding interactions.

US8501923 describes scaffolded self-assembly of nucleic acid strands to create arbitrary shapes and patterns of nucleic acids using long DNA scaffold strands in combination with a large number of short strands. This allows the creation of nanostructures in high yield without any requirement for specially designed scaffolds or extensive purification.

The article "Diverse and robust molecular algorithms using reprogrammable DNA self-assembly" by Damien Woods, David Doty et. al. (Nature Vol 567, 2019) describes a DNA tile set that can be reprogrammed to implement a wide variety of algorithms using molecular self-assembly at a fixed holding temperature.

The article "Parallel molecular computation on digital data stored in DNA" by Boya Wang et. al. (PNAS Vol. 120 No. 37, 2023) describes molecular computing using strand displacement reactions to algorithmically modify data in a parallel manner (SIMD||DNA), based on toehold-mediated strand displacement (TMSD). This merges DNA storage with DNA computing, and proposes entirely molecular algorithms for parallel manipulation of digital information preserved in DNA based on DNA strand displacement reactions. This allows computations directly on the storage medium, and thereby parallel processing of large volumes of data.

### PROBLEMS WITH THE PRIOR ART

Prior art DNA computing systems are not thermodynamically favoured. The overwhelming majority of large multi-tile configurations are not the target output. For both algorithmic self-assembly systems and TMSD systems, algorithmic errors and off-seeded growth, also called spurious nucleation, can occur.

Moreover, the target output of prior art molecular computing systems is out-of-equilibrium, meaning that they suffer from errors such as leaks in strand displacement circuits. This means that the results will not be stable.

There is thus a need for improved methods for making molecular computations.

### SUMMARY

The above-described problem is addressed by the claimed method for making molecular computations. The method preferably comprises: i) providing a mixture comprising a scaffold, comprised of a long information-encoding molecule or strand, such as e.g. a long polynucleotide strand or a long amino acid sequence, and comprising N binding domains, each binding domain constituting a scaffold position; ii) designing a set of computing tiles to drive a desired computation, the set of computing tiles comprising at least N different computing tile types, wherein the computing tile types are selected to be used for the computation, in such a way that a target output has a higher probability of being reached than the probability for any other potential output, where each computing tile is comprised of a short information-encoding molecule or strand, such as e.g. a short polynucleotide or a short amino acid sequence, and comprises a bottom position domain and at least one compute domain, wherein for each computing tile, the bottom position domain is arranged to bind directly to a matching scaffold position on the scaffold with a first binding strength, and to the compute domains of other computing tiles with a set of second binding strengths, which are each weaker than the first binding strength; iii) adding the designed set of computing tiles to the mixture; iv) allowing bottom position domains of computing tiles to bind to scaffold positions, until all scaffold positions required for the computation have been filled; v) allowing replacement of all mismatched computing tiles, based on correct compute domain bindings being enthalpically favoured over incorrect compute domain bindings (algorithmic errors); and vi) reaching an output configuration. The target output may even have a higher probability of being reached than the sum of the probabilities for all other potential outputs.

This method avoids the need for extra error-correction subsystems, since the molecular computations naturally evolve to the target output at equilibrium.

In embodiments, the output of the molecular computation is stored. The method may thereby be used also for storage of data created by a molecular computation, e.g. to enable further computations on the stored data at a later stage.

In embodiments, the designing comprises ensuring that there for each computing tile type is a selected concentration of computing tiles in the mixture, the selected concentration ensuring that there is an excess of computing tiles of the computing tile type in the mixture. This ensures that there will always be a suitable computing tile of the correct computing tile type available to bind to the scaffold position, and also makes the method less vulnerable to impurities in the computing tiles. All computing tiles of the same computing tile type are preferably substantially identical.

In embodiments, the method further comprises heating the mixture to release all bindings. The allowing of bottom position domains of computing tiles to bind to scaffold positions, and the allowing of replacement of all mismatched computing tiles, then preferably take place as the mixture cools. The reaching of an output configuration will in this case typically take place when the mixture has cooled. This allows for a much quicker computation. It is however not necessary to heat and cool the mixture, it is perfectly possible to let the mixture instead have a constant temperature. In this case, a suitable temperature should be selected (the temperature may e.g. not be so high that the bindings are released).

In embodiments, the providing comprises arranging the binding domains on the scaffold to be unique. However, many or all of the binding domains on the scaffold may alternatively be similar to each other.

In embodiments, the providing comprises arranging the binding domains on the scaffold to all have approximately the same length and/or binding strength. However, the binding domains are not necessarily fixed domains on the scaffold. They may even in embodiments have a variable length and/or binding strength.

In embodiments, the scaffold further comprises an additional binding domain in the form of an anchor position, and the set of computing tiles further comprises an anchor tile comprising a bottom position domain and an anchoring compute domain. The allowing may then further comprise allowing the bottom position domain of the anchor tile to bind to the anchor position. The anchor tile does not have to be added at the beginning of the computation, it is possible to add it at a later stage (especially in embodiments where the mixture is not heated), but this will make the computation slower.

In embodiments, further computations are allowed by resetting the mixture by adding at least one blocking computing tile to the mixture, wherein each added blocking computing tile is arranged to bind to the bottom position domain and to at least one compute domain of a selected computing tile, thereby blocking the selected computing tile from being used in the computation. Steps ii-vi may then be repeated. The mixture may be heated to release all bindings before adding the blocking computing tile, but this is not necessary - it is perfectly possible to let the mixture instead have a constant temperature, even during resetting of the mixture.

In embodiments, the providing further comprises providing a reporting tile, preferably comprising a fluorophore or a quencher, i.e. having an attached fluorophore/quencher "label", in the mixture, wherein the reporting tile, directly or via an intermediate tile, is arranged to bind to a reporting position, which is a further scaffold position immediately following the final scaffold position that is required for the computation. The designing further comprises designing a result indicating tile, preferably comprising a fluorophore or a quencher, i.e. having an attached fluorophore/quencher "label", to bind to the target final compute domain, and the adding further comprises adding the result indicating tile to the mixture. The method may then further comprise reporting the outcome of the molecular computation by reporting the detected fluorescence signal or level caused by the energy transfer between the reporting tile and the result indicating tile, when the reporting tile, directly or via the intermediate tile, has bound to the reporting position, and the result indicating tile has bound to the actual final compute domain. This energy transfer depends on the distance between the fluorophore/quencher on the reporting tile and the fluorophore/quencher on the result indicating tile, which depends on to what extent the result indicating tile has bound to the actual final compute domain. This indicates to what extent the actual final compute domain corresponds to the target final compute domain.

At least one of the reporting tile and the result indicating tile preferably comprises a fluorophore. In embodiments where both the reporting tile and the result indicating tile comprises a fluorophore, the reporting may use multi-level Fluorescence Resonance Energy Transfer (FRET).

Detection of more than two different outcomes may comprise detecting variations in the fluorescence signal or level at one or more selected temperatures. The one or more selected temperatures are preferably temperatures at which multi-level fluorescence takes place.

In embodiments, the designing comprises designing the second binding strengths in the set of second binding strengths to be approximately equal to each other.

The above-described problem is also addressed by the claimed arrangement for making molecular computations. The arrangement preferably comprises: a mixture comprising a scaffold, comprised of a long information-encoding molecule or strand, such as e.g. a long polynucleotide strand or a long amino acid sequence, and comprising N binding domains, each binding domain constituting a scaffold position; a set of computing tiles, which has been designed to drive a desired computation, wherein the designed set of computing tiles comprises at least N different computing tile types, which have been selected to be used for the computation in such a way that a target output has a higher probability of being reached than the probability for any other potential output, each computing tile comprised of a short information-encoding molecule or strand, such as e.g. a short polynucleotide or a short amino acid sequence, and comprising a bottom position domain and at least one compute domain, wherein for each computing tile, the bottom position domain is arranged to bind directly to a matching scaffold position on the scaffold with a first binding strength, and to the compute domains of other computing tiles with a set of second binding strengths, which are each weaker than the first binding strength. The arrangement is preferably configured to: allow bottom position domains of computing tiles to bind to scaffold positions until all scaffold positions have been filled; and allow replacement of all mismatched computing tiles, based on correct compute domain bindings being arranged to be enthalpically favoured over incorrect compute domain bindings, thereby allowing an output configuration to be reached. The target output may even have a higher probability of being reached than the sum of the probabilities for all other potential outputs.

This arrangement avoids the need for extra error-correction subsystems, since the molecular computations naturally evolve to the target output at equilibrium.

In embodiments, the output of the molecular computation is stored. The arrangement may thereby be used also for storage of data created by a molecular computation, e.g. to enable further computations on the stored data at a later stage.

In embodiments, there is for each computing tile type a selected concentration of computing tiles in the mixture, the selected concentration ensuring that there is an excess of computing tiles of the computing tile type in the mixture. This ensures that there will always be a suitable computing tile of the correct computing tile type available to bind to the scaffold position, and also makes the arrangement less vulnerable to impurities in the computing tiles. All computing tiles of the same computing tile type are preferably substantially identical.

In embodiments, the arrangement is preferably configured to heat the mixture to release all bindings. The allowing of bottom position domains of computing tiles to bind to scaffold positions, and the allowing of replacement of all mismatched computing tiles, then preferably take place as the mixture cools. The reaching of an output configuration will in this case typically take place when the mixture has cooled. This allows for a much quicker computation. It is however not necessary to heat and cool the mixture, it is perfectly possible to let the mixture instead have a constant temperature. In this case, a suitable temperature should be selected (the temperature may e.g. not be so high that the bindings are released).

In embodiments, the binding domains on the scaffold are unique. However, many or all of the binding domains on the scaffold may alternatively be similar to each other.

In embodiments, the binding domains on the scaffold all have approximately the same length and/or binding strength. However, the binding domains are not necessarily fixed domains on the scaffold. They may even in embodiments have a variable length and/or binding strength.

In embodiments, the scaffold further comprises an additional binding domain in the form of an anchor position, and the set of computing tiles further comprises an anchor tile comprising a bottom position domain and an anchoring compute domain, and the bottom position domain of the anchor tile is configured to bind to the anchor position. The anchor tile does not have to be added at the beginning of the computation, it is possible to add it at a later stage (especially in embodiments where the mixture is not heated), but this will make the computation slower.

In embodiments, the arrangement is configured to allow further computations by resetting the mixture by at least one blocking computing tile being added to the mixture, wherein each added blocking computing tile is arranged to bind to the bottom position domain and to at least one compute domain of a selected computing tile, thereby blocking the selected computing tile from being used in the computation. One or more new computing tiles may then be added to the mixture, to allow a new computation to be made. The mixture may be heated to release all bindings before adding the blocking computing tile, but this is not necessary - it is perfectly possible to let the mixture instead have a constant temperature, even during resetting of the mixture.

In embodiments, the mixture further comprises a reporting tile, preferably comprising a fluorophore or a quencher, i.e. having an attached fluorophore/quencher "label", where the reporting tile, directly or via an intermediate tile, is designed to bind to a reporting position, which is a further scaffold position immediately following the final scaffold position that is required for the computation, and a result indicating tile, preferably comprising a fluorophore or a quencher, i.e. having an attached fluorophore/quencher "label", designed to bind to the target final compute domain. The arrangement may then be configured to report the outcome of the molecular computation by reporting the detected fluorescence signal or level caused by the energy transfer between the reporting tile and the result indicating tile, when the reporting tile, directly or via the intermediate tile, has bound to the reporting position, and the result indicating tile has bound to the actual final compute domain. This energy transfer depends on the distance between the fluorophore/quencher on the reporting tile and the fluorophore/quencher on the result indicating tile, which depends on to what extent the result indicating tile has bound to the actual final compute domain. This indicates to what extent the actual final compute domain corresponds to the target final compute domain.

At least one of the reporting tile and the result indicating tile preferably comprises a fluorophore. In embodiments where both the reporting tile and the result indicating tile comprises a fluorophore, the reporting may use multi-level FRET.

Detection of more than two different outcomes may comprise detecting variations in the fluorescence signal or level at one or more selected temperatures. The one or more selected temperatures are preferably temperatures at which multi-level fluorescence takes place.

In embodiments, the second binding strengths in the set of second binding strengths are approximately equal to each other.

A method for determining the status of a target molecule in a molecular mixture is also claimed. The method preferably comprises: adding a reporting strand, which may bind to an intermediate strand, to a molecular mixture, wherein the reporting strand preferably comprises a fluorophore or a quencher, i.e. has an attached fluorophore/quencher "label"; designing a result indicating strand to bind to a predetermined extent to the target molecule, wherein the result indicating strand preferably comprises a fluorophore or a quencher, i.e. has an attached fluorophore/quencher "label"; adding the result indicating strand to the molecular mixture; detecting the fluorescence signal or level caused by the energy transfer between the reporting strand and the result indicating strand; and determining the status of the target molecule based on a comparison of the fluorescence signal with a predetermined range corresponding to a predetermined status of the target molecule. The energy transfer depends on the distance between the fluorophore/quencher on the reporting strand and the fluorophore/quencher on the result indicating strand.

An arrangement for determining the status of a target molecule in a molecular mixture is also claimed. The arrangement preferably comprises a molecular mixture comprising a target molecule, a reporting strand, which may bind to an intermediate strand, wherein the reporting strand preferably comprises a fluorophore or a quencher, i.e. has an attached fluorophore/quencher "label", and a result indicating strand, which has been designed to bind to a predetermined extent to the target molecule, wherein the result indicating strand preferably comprises a fluorophore or a quencher, i.e. has an attached fluorophore/quencher "label". The arrangement is preferably configured to detect the fluorescence signal or level caused by the energy transfer between the reporting strand and the result indicating strand, and determine the status of the target molecule based on a comparison of the fluorescence signal with a predetermined range corresponding to a predetermined status of the target molecule. The energy transfer depends on the distance between the fluorophore/quencher on the reporting strand and the fluorophore/quencher on the result indicating strand.

At least one of the reporting strand and the result indicating strand preferably comprises a fluorophore. In embodiments where both the reporting strand and the result indicating strand comprises a fluorophore, the reporting may use multi-level FRET.

In embodiments, the status of the target molecule comprises to what extent the result indicating strand binds to the target molecule.

In embodiments, the target molecule is a part of a predetermined set of different molecules, the result indicating strand is arranged to bind to different extents to each molecule in the predetermined set of different molecules, and the status of the target molecule comprises the presence of the target molecule in the molecular mixture.

In embodiments, the target molecule is a scaffold, the reporting strand is designed to bind to a reporting position on the scaffold, and the result indicating strand is designed to bind to a predetermined extent to a scaffold position close to the reporting position.

In embodiments, the reporting strand binds to an intermediate strand, which is designed to bind to the target molecule.

In embodiments, the target molecule comprises a target final compute domain of a computing tile.

Detection of more than two different outcomes may comprise detecting variations in the fluorescence signal or level at one or more selected temperatures. The one or more selected temperatures are preferably temperatures at which multi-level fluorescence takes place.

This type of multi-level reporting mechanism may be used a variety of situations where two or more DNA/RNA/amino strands are co-located. It may be used in the above described method and/or arrangement, but it may also have a more general use for determining the status of a target molecule in a molecular mixture. The mechanism may thus have application also in other types of molecular systems, such as e.g. systems for molecular biology (e.g. PCR), synthetic biology, and/or DNA/RNA/amino acid nanostructures.

The binding domains are not necessarily fixed domains on the scaffold. They may in embodiments have a variable length and/or binding strength, depending e.g. on the bottom position domain of the computing tile that binds to the binding domain on the scaffold. The positions of the binding domains on the scaffold may thus also be variable. In such embodiments, it may only be possible to determine the actual length and/or binding strength and position of a binding domain on the scaffold once a bottom position domain of a computing tile has bound to the binding domain on the scaffold.

A tile or strand may be defined as an information-encoding molecule, and may be either a polynucleotide strand or an amino acid sequence.

A "long" information-encoding molecule or strand may be defined as an information-encoding molecule or strand that is at least five times the length of a "short" information-encoding molecule or strand. In most embodiments, the "long" information-encoding molecule or strand will be more than ten times the length of the "short" information-encoding molecule or strand, and it may even be a hundred or a thousand times the length of the "short" information-encoding molecule or strand.

The first binding strength may be defined as a "scaffold position binding strength", and the second binding strength may be defined as a "compute domain binding strength".

A molecular computation may thus be "programmed" by selecting the computing tile types to use for the computation. The compute domains of the computing tiles encode both the input and the program, i.e. both data and program bits. Running a program with a different input is effected by changing the set of computing tiles, and changing the program is also effected by changing the set of computing tiles. In such molecular computations, there will always be multiple kinetic pathways leading to the output.

The scope of the invention is defined by the claims, which are incorporated into this section by reference. A more complete understanding of embodiments of the invention will be afforded to those skilled in the art, as well as a realization of additional advantages thereof, by a consideration of the following detailed description of one or more embodiments. Reference will be made to the appended sheets of drawings that will first be described briefly.

### BRIEF DESCRIPTION OF THE DRAWINGS

Fig. 1a schematically illustrates an arrangement for making molecular computations, in accordance with one or more embodiments described herein.
Fig. 1b schematically illustrates the three key principles allowing the target output to be energetically favourable.
Fig. 1c schematically illustrates a target output of a molecular computation, in accordance with one or more embodiments described herein.
Fig. 2 schematically illustrates the tile bind operation as a hybridisation of a computing tile to a scaffold position, in accordance with one or more embodiments described herein.
Fig 3a-b schematically illustrate reporting mechanisms, in accordance with embodiments described herein.
Fig. 3c schematically illustrates temperature curves enabling the detection of multi-level fluorescence, in accordance with one or more embodiments described herein.
Fig. 4 schematically illustrates a general compilation scheme from a Finite State Machine into computing tiles, in accordance with one or more embodiments described herein.
Fig. 5 schematically illustrates the programming of a Finite State Machine, in accordance with one or more embodiments described herein.
Figs. 6a-c schematically illustrate an example ADDITION program, in accordance with one or more embodiments described herein.
Figs. 7a-b schematically illustrate a molecular computation, in accordance with one or more embodiments described herein.
Fig. 8 schematically illustrates a method for making molecular computations, in accordance with one or more embodiments described herein.
Fig. 9 schematically illustrates a method for detecting the co-location of molecules, in accordance with one or more embodiments described herein.

Embodiments of the present disclosure and their advantages are best understood by referring to the detailed description that follows. It should be appreciated that like reference numerals are used to identify like elements illustrated in one or more of the figures.

### DETAILED DESCRIPTION

Prior art molecular computers will typically have a target output which is out-of-equilibrium, because of leaks in the strand displacement circuit. This means that the results will not be stable. The present disclosure proposes thermodynamically favoured molecular computing, which applies thermodynamic penalties to logical errors in the strand displacement process, by correct compute domain bindings being enthalpically favoured over incorrect compute domain bindings (algorithmic errors). In this way, logical errors are automatically repaired by the system going to equilibrium. This means that there will be no need for error correction, since there will be a natural evolution to the target output at equilibrium. The target output is highly favoured, thereby outcompeting all other output configurations.

What is proposed herein is a thermodynamically favoured, programmable, scaffolded molecular computer, described in the form of a scaffolded DNA computer (SDC). An N-position *I*-bit SDC may have a one-dimensional scaffold structure with N binding domains called scaffold positions. A set of computing tiles may be assigned to each scaffold position. The computing tiles are preferably of different computing tile types, where all computing tiles of the same computing tile type are substantially identical.

The left and right sides of a computing tile are herein called "compute domains", and the bottom side is herein called a "bottom position domain". A computing tile typically has two compute domains. During a computation, each computing tile binds by its bottom position domain to a matching scaffold position. Information is processed by the binding of adjacent matching compute domains. Mismatching adjacent compute domains are permitted, but are an algorithmic error, implying an enthalpic cost that will be resolved by the future replacement of one or both tiles. The molecular process of choosing the correct tile per position executes the computation.

A specific computing tile called an anchor tile may be used to initiate the computation. The anchor tile binds with its bottom position domain to an additional binding domain on the scaffold in the form of an anchor position, and to the compute domain of a computing tile with an anchoring compute domain. The anchor tile is preferably designed in such a way that a target output has a higher probability of being reached than the probability for any other potential outputs. The target output may even have a higher probability of being reached than the sum of the probabilities for all other potential outputs.

SDC computation avoids the need for extra error-correction subsystems by naturally evolving to the target output at equilibrium. The SDC target output is highly favoured, outcompeting exponentially many other output configurations. Finally, the SDC design preferably has highly parallel kinetics, with multiple potential pathways to the output, a novel thermodynamic form of molecular computing that, unlike previous pathway-driven work, does not seek to tightly control kinetics.

Three principles ensure that the target output is thermodynamically favoured:
1) There are at least as many computing tile types as scaffold positions required for the computation, thereby ensuring that each scaffold position gets occupied by a computing tile. There is preferably also a concentration excess (stoichiometric excess) over scaffold positions for the computing tiles of each computing tile type. This ensures that there will always be a suitable computing tile of the correct computing tile type available to bind to the scaffold position, and also makes the SDC less vulnerable to impurities in the computing tiles.
2) Correct compute domain bindings are enthalpically favoured over algorithmic errors.
3) Compute domain binding strength is weaker than scaffold position binding strength.

A molecular computation may thus be "programmed" by selecting the computing tile types to use for the computation, e.g. from a pool of computing tiles. The compute domains of the computing tiles encode both the input and the program, i.e. both data and program bits. Running a program with a different input is effected by changing the set of computing tiles, and changing the program is also effected by changing the set of computing tiles. In such molecular computations, there will always be multiple kinetic pathways leading to the output.

The term that an output configuration is "enthalpically favoured", "thermodynamically favoured" or "energetically favoured" defines that the output configuration is the most favoured output configuration, which will eventually be reached in any computation. This claim can for specific computing programs be mathematically proven.

The binding domains are not necessarily fixed domains on the scaffold. They may in embodiments have a variable length and/or binding strength, depending e.g. on the bottom position domain of the computing tile that binds to the binding domain on the scaffold. The position of the binding domains on the scaffold may thus also be variable. In such embodiments, it may only be possible to determine the actual length and/or binding strength and position of a binding domain on the scaffold once a bottom position domain of a computing tile has bound to the binding domain on the scaffold.

The present disclosure relates generally to arrangements and methods for making molecular computations. Embodiments of the disclosed solution are presented in more detail in connection with the figures.

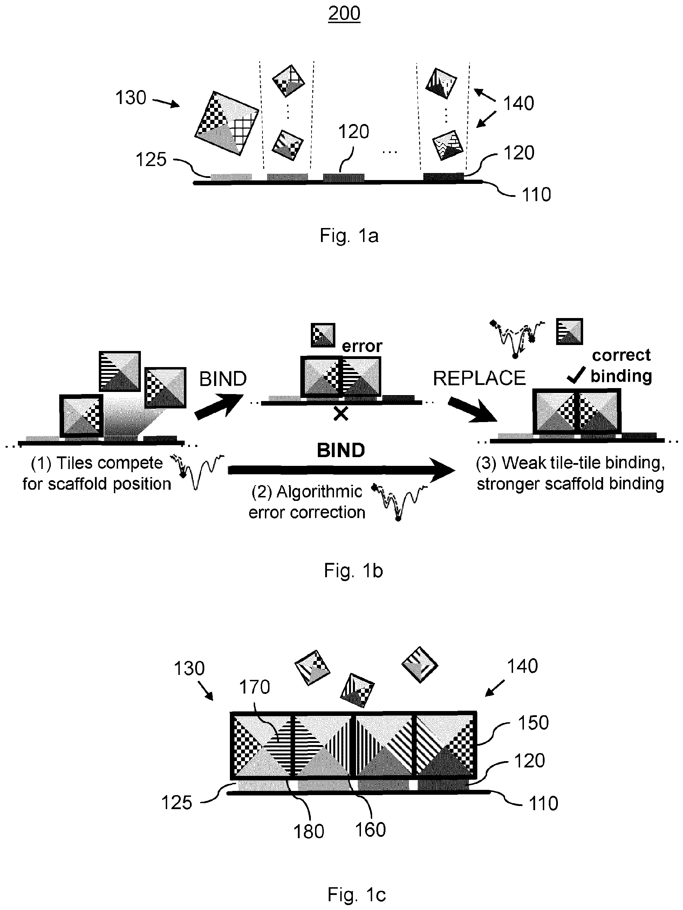

Fig. 1a schematically illustrates an SDC 200 comprising a scaffold 110 comprised of a long information-encoding molecule or strand, such as e.g. a long helical single stranded polynucleotide strand or a long amino acid sequence. The scaffold 110 comprises N binding domains, each binding domain constituting a scaffold position 120 required for the computation. The scaffold 110 may also comprise an additional binding domain in the form of an anchor position 125. The binding domains are preferably unique, but many or all of the binding domains may alternatively be similar to each other.

The schematically illustrated SDC further comprises a plurality of computing tiles 140, one of which being an anchor tile 130, where each computing tile 140 is comprised of a short information-encoding molecule or strand, such as e.g. a short polynucleotide or a short amino acid sequence. The computing tiles 140 are preferably of different computing tile types, where all computing tiles 140 of the same computing tile type are substantially identical. Each computing tile 140 comprises a bottom position domain 160 and at least one compute domain 150, wherein the bottom position domain is arranged to bind directly to a matching scaffold position 120 on the scaffold 110, and the compute domains 150 are arranged to bind to compute domains 150 of other computing tiles 140. Computing tiles 140 are schematically illustrated as unit-sized squares with patterns on both their compute domains 150 and their bottom position domains 160. A computing tile 140 typically has two compute domains 150. There are preferably at least N different computing tile types, so that computing tiles 140 of different computing tile types compete for the scaffold positions 120. There is preferably also a concentration excess (stoichiometric excess) over scaffold positions 140 for the computing tiles 140 of each computing tile type. This ensures that there will always be a suitable computing tile 140 of the correct computing tile type available to bind to the scaffold position 120, and also makes the SDC less vulnerable to impurities in the computing tiles 140. All computing tiles 140 of the same computing tile type are preferably substantially identical.

An anchor tile 130 may be used to initiate the computation. The anchor tile 130 binds to the anchor position 125 on the scaffold 110 with its bottom position domain 180, and to the compute domain 150 of a computing tile 140 with its anchoring compute domain 170. The anchor tile 130 is preferably designed in such a way that a target output has a higher probability of being reached than the probability for any other potential outputs. The target output may even have a higher probability of being reached than the sum of the probabilities for all other potential outputs. The anchor tile 130 does not have to be added at the beginning of the computation, it is possible to add it at a later stage (especially in embodiments where the mixture 100 is not heated), but this will make the computation slower.

Heating the mixture 100 to release all bindings, and allowing the computation to take place as the mixture 100 cools, allows for a much quicker computation. It is however not necessary to heat and cool the mixture, it is perfectly possible to let the mixture instead have a constant temperature. In this case, a suitable temperature should be selected (the temperature may e.g. not be so high that the bindings are released).

An N-position SDC 200 has a one-dimensional scaffold structure 110 with N scaffold positions 120. A set of computing tiles 140 of a computing tile type is preferably assigned to each scaffold position 120. During a computation, the bottom position domain 180 of the anchor tile 130 binds to the anchor position 125, and the other computing tiles 140 bind by their bottom position domain 160 to matching scaffold positions 120. Information is processed by the binding of adjacent matching compute domains 150. Mismatching adjacent compute domains 150 are permitted, but are an algorithmic error, implying an enthalpic cost that will be resolved by the future replacement of one or both computing tiles 140. SDC programming may be defined as selecting a set of computing tiles 140 that determine the left-to-right instructions to be computed per scaffold position 120. A desired program is mapped to a set of computing tiles 140, where the compute domains 150 encode both the input and the program, i.e. both data and program bits. Running a program with a different input is effected by changing the set of computing tiles 140, and changing the program is also effected by changing the set of computing tiles 140. In such molecular computations, there will always be multiple kinetic pathways leading to the output.

A typical deterministic computation has a single anchor tile 130 at the anchor position 125, and multiple competing computing tiles 140 of different computing tile types at the other scaffold positions 120, where only one computing tile type is correct per scaffold position 120. The molecular process of choosing the correct computing tile type per scaffold position 120 executes the computation. A sequence of computing tiles 140 bound to matching positions on the scaffold 110 is a valid computation, or target output.

Fig. 1b schematically illustrates the three key principles yielding the target output being energetically favourable:
1) There is at least the same amount of computing tile types as scaffold positions 120, thereby ensuring that each scaffold position 120 gets occupied by a matching computing tile 140. Further, inspired by DNA origami, computing tiles 140 of each computing tile type are preferably at a concentration excess (typically 10×) over scaffold positions 120. This ensures that there will always be a suitable computing tile 140 of the correct computing tile type available to bind to the scaffold position 120, and also makes the SDC less vulnerable to impurities in the computing tiles 140.
2) Correct compute domain bindings are enthalpically favoured over algorithmic errors, thereby ensuring an automatic algorithmic error correction.
3) The compute domain binding strength is weaker than the scaffold position binding strength, thereby ensuring replacement to create correct bindings.

For each computing tile 140, the bottom position domain 160 is thus arranged to bind directly to a binding domain (a scaffold position 120) on the scaffold 110 with a first binding strength (the scaffold position binding strength), and to the compute domains 150 of other computing tiles 140 with a set of second binding strengths (the compute domain binding strengths), which are each weaker than the first binding strength (the scaffold position binding strength). This is a key feature of the disclosed molecular computing concept.

Fig. 1c schematically illustrates a target output of the SDC, in the form of a thermodynamically favoured output.

The SDC is preferably arranged to be renewable and allow further computations to be made. This may be effected by resetting the mixture 100 by adding at least one blocking computing tile to the mixture 100, wherein the blocking computing tile is arranged to bind to the bottom position domain 160, 180 and to at least one compute domain 150, 170 of a selected computing tile 130, 140, thereby blocking the selected computing tile 130, 140 from being used in the computation, by preventing the bottom position domain 160, 180 of the selected computing tile 130, 140 from binding to the scaffold position 120, 125 of the scaffold 110, and at least one compute domain 150, 170 from binding to another compute domain 150. The selected computing tile may e.g. be the anchor tile 130, but any number of computing tiles may in this way be blocked from being used in a computation. One or more new computing tiles 140, e.g. a new anchor tile 130, may then be added to the mixture 100, to allow a new computation to be made. The mixture 100 may be heated to release all bindings before adding the at least one blocking computing tile, but this is not necessary - it is perfectly possible to let the mixture 100 instead have a constant temperature, even during resetting of the mixture 100.

The SDC enables robust, fast and renewable thermo-dynamically favoured molecular computing. Intuition suggests equilibrium computation to be imprecise, since any target output configuration competes with exponentially many off-target configurations. However, the proposed thermodynamic design principles, that leverage theory and simulation, engineer the predicted target Minimum-Free-Energy (MFE) output to have probability approaching 1.0, outcompeting all other configurations. Thus, the SDC is robust by design. Unlike kinetically-driven molecular computers, no explicit error correction techniques (such as e.g. redundancy (proofreading), scaleup (leakless), mismatches, GC-ends or clamps) are needed. There is no need for compute-strand purification, long temperature holds, multi-step manual protocols, day-long temperature holds within a precisely-calibrated 0.3°C window, or early experiment termination to avoid leak. Finally, unlike previous computing systems, the SDC is concentration-robust, inheriting the beautiful principle from DNA origami of having staple (compute) strands in large concentration excess (stoichiometric excess) over the binding domains on the scaffold.

The tile base model described herein is intentionally oversimplified by having unit-strength binding, no notion of temperature, an implicitly rigid structure, and ignoring entropy.

What is shown in Figs. 1a-c is an arrangement 200 for making molecular computations, the arrangement 200 comprising: a mixture 100 comprising a scaffold 110 comprised of a long information-encoding molecule or strand, such as e.g. a long polynucleotide strand or a long amino acid sequence, and comprising N binding domains, each binding domain constituting a scaffold position 120 required for the computation; and a set of computing tiles which has been designed to drive the computation, wherein the set of computing tiles comprises at least N different computing tile types, which have been selected to be used for the computation in such a way that a target output has a higher probability of being reached than the probability for any other potential output. Each computing tile 140 is comprised of a short information-encoding molecule or strand, such as e.g. a short polynucleotide or a short amino acid sequence, and comprises a bottom position domain 160 and at least one compute domain 150, wherein for each computing tile 140, the bottom position domain 160 is arranged to bind directly to a matching scaffold position 120 on the scaffold 110 with a first binding strength, and to the compute domains 150 of other computing tiles 140 with a set of second binding strengths, which are each weaker than the first binding strength. The scaffold 110 may in embodiments also comprise an anchor position 125, and the set of computing tiles may comprise an anchor tile 130 comprising a bottom position domain 180 and an anchoring compute domain 170. The arrangement 200 is configured to: allow the bottom position domain 180 of the anchor tile 130 (if used) to bind to the anchor position 125, and bottom position domains 160 of computing tiles 140 to bind to scaffold positions 120, until all scaffold positions 120 required for the computation have been filled; and allow replacement of all mismatched computing tiles 140, based on correct compute domain bindings being arranged to be enthalpically favoured over incorrect compute domain bindings, thereby allowing an output configuration to be reached. The target output may even have a higher probability of being reached than the sum of the probabilities for all other potential outputs.

This arrangement 200 avoids the need for extra error-correction subsystems, since the molecular computations naturally evolve to the target output at equilibrium.

In embodiments, there is for each computing tile type a selected concentration of computing tiles 140 in the mixture 100, the selected concentration ensuring that there is an excess of computing tiles 140 of the computing tile type in the mixture 100. This ensures that there will always be a suitable computing tile 140 of the correct computing tile type available to bind to the scaffold position 120, and also makes the arrangement 200 less vulnerable to impurities in the computing tiles 140. All computing tiles 140 of the same computing tile type are preferably substantially identical.

In embodiments, the binding domains on the scaffold are unique. However, many or all of the binding domains on the scaffold may alternatively be similar to each other. The binding domains on the scaffold may all have the same length and/or binding strength.

The binding domains are not necessarily fixed domains on the scaffold 110. They may in embodiments have a variable length and/or binding strength, depending e.g. on the bottom position domain 160 of the computing tile 140 that binds to the binding domain on the scaffold. The positions of the binding domains on the scaffold may thus also be variable. In such embodiments, it may only be possible to determine the actual length and/or binding strength and position of a binding domain on the scaffold once a bottom position domain 160 of a computing tile 140 has bound to the binding domain on the scaffold.

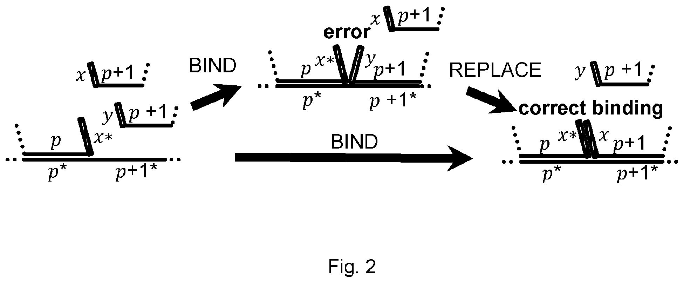

If the bottom position domains 160 of a computing tile 140 incorrectly binds at a scaffold position 120, there is preferably a computing tile displacement pathway to discard the incorrect computing tile 140. This is schematically illustrated in Fig. 2, where two computing tiles 140 compete for the same scaffold position 110. Fig. 2 schematically illustrates the tile bind operation as a hybridisation of a computing tile 140 binding to a scaffold position 120 and any adjacent matching compute domains 150. In Fig. 2, the kinetic details of the replace pathway are intentionally left unspecified: it can be any pathway that swaps scaffold-binding computing tile 140. There may be many plausible, temperature-dependent, pathways. The replacement mechanism may thus be any reasonable molecular mechanism that results in the replacement of a mismatched computing tile for another computing tile. The replacement mechanism could e.g. be a 1-step replacement or a bind/unbind mechanism.

This laissez-faire design approach stands in contrast to DNA computing using toehold-mediated strand displacement where precise, and often unique, kinetic pathways are engineered. The combination of thermodynamic favourability and multiple plausible kinetic pathways enables a straightforward temperature anneal to overcome kinetic traps, unlike carefully optimised prior art systems. To avoid unintended hairpin formations, each 3-bit sequence may have two distinct compute domains 150.

### Multi-level fluorescence

The outcome of a molecular computation may be detected by adding a reporting tile 350 to the mixture 100. The reporting tile 350 may be arranged to, directly or via an intermediate tile 330, bind to a reporting position 320, which is a further scaffold position immediately following the final scaffold position 120 required for the computation. The reporting tile 350 preferably comprises a fluorophore or a quencher, i.e. has an attached fluorophore/quencher "label". If a result indicating tile 310, preferably comprising a fluorophore or a quencher, i.e. having an attached fluorophore/quencher "label", is designed to bind to the target final compute domain 150, the output of the molecular computation may be determined by detecting the fluorescence signal or level caused by the energy transfer between the reporting tile 350 and the result indicating tile 310, when the reporting tile 350, directly or via the intermediate tile 330, has bound to the reporting position 320, and the result indicating tile 310 has bound to the actual final compute domain 150. This energy transfer depends on the distance between the fluorophore/quencher on the reporting tile 350 and the fluorophore/quencher on the result indicating tile 310, which depends on to what extent the result indicating tile 310 has bound to the actual final compute domain 150. This indicates to what extent the actual final compute domain 150 corresponds to the target final compute domain 150.

At least one of the reporting tile 350 and the result indicating tile 310 preferably comprises a fluorophore. If both the reporting tile 350 and the result indicating tile 310 comprises a fluorophore, the reporting may use multi-level FRET.

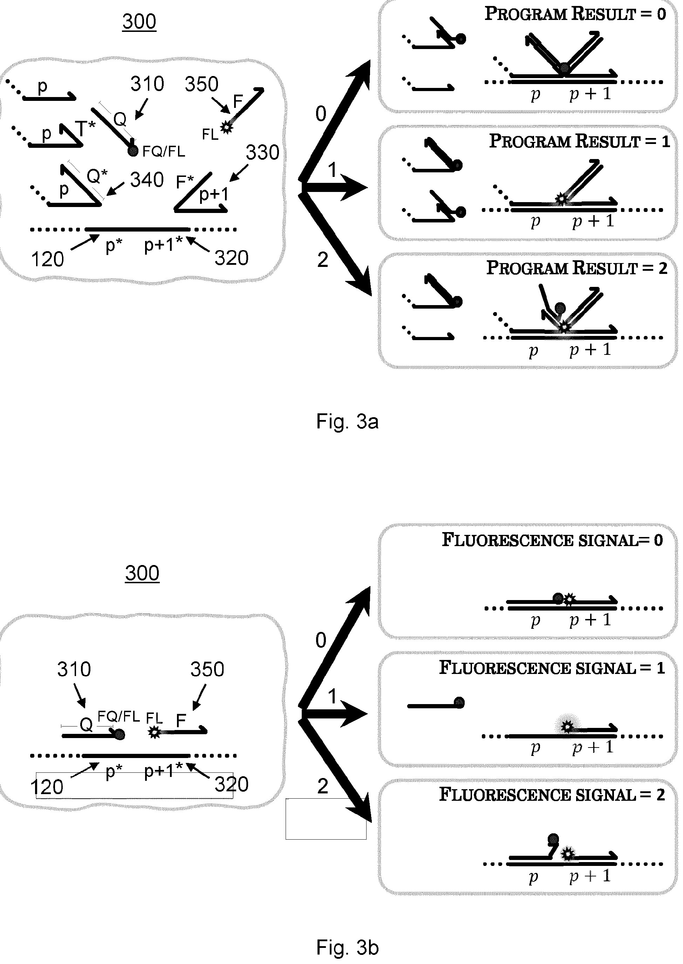

Ternary reporting mechanisms are schematically illustrated by the arrangements 300 in Figs 3a-b. Prior art arrangements have only been able to report binary outcomes: 0 (full quench) or 1 (no quench). However, the inventors have realized that it is possible to determine more than two different outcomes by detecting variations in the fluorescence signal or level. This enables the detection of multi-level fluorescence, e.g. when the actual final compute domain 150 is not a perfect complement to the result indicating tile 310, and the target output has thus not been fully reached. If it can be detected that parts of the actual final compute domain 150 is a complement to parts of the result indicating tile 310, it can be determined to what extent the actual output corresponds to the target output.

In the arrangement 300 in Fig. 3a, three potential final compute domains 150 are illustrated to the left, in the three potential molecules 340.

If the result indicating tile 310 binds to the top one, there will be no part of the compute domain 150 that is a complement any part of the result indicating tile 310, and therefore no binding between the result indicating tile 310 and the compute domain 150. There is then no energy transfer between the reporting tile 350 and the result indicating tile 310, and thus the reporting result is 1.

If the result indicating tile 310 binds to the middle one, there will be some part of the compute domain 150 that is a complement some part of the result indicating tile 310, causing a partial binding between the result indicating tile 310 and the compute domain 150. There is then some energy transfer between the reporting tile 350 and the result indicating tile 310, and thus the reporting result is 2.

If the result indicating tile 310 binds to the bottom one, the compute domain 150 is a complement the result indicating tile 310, causing full binding between the result indicating tile 310 and the compute domain 150. There is then full energy transfer between the reporting tile 350 and the result indicating tile 310, and thus the reporting result is 0.

By detecting to what extent parts of the actual final compute domain 150 is a complement to parts of the result indicating tile 310, it can thus be determined to what extent the actual output corresponds to the target output. The reporting result (for the situation where the result indicating tile 310 comprises a quencher): 0 (full quench), 1 (no quench) or 2 (partial quench) is illustrated to the right in Fig. 3a. A 5-strand reporter complex with 5' fluorophore and 3' quencher may e.g. be used for the reporting.

This type of multi-level reporting mechanism may be used in a variety of situations where two or more DNA/RNA/amino strands are co-located. It may be used for detecting and reporting the outcome of a molecular computation, but it may also have a more general use for determining the status of a target molecule/strand/tile 340 in a molecular mixture 100. The result indicating strand/tile 310 would then be designed to bind to a predetermined extent to a target molecule/strand/tile 340 in the molecular mixture, and the status of the target molecule/strand/tile 340 would be detected by detecting the fluorescence signal or level caused by the energy transfer between the reporting strand/tile 350 and the result indicating strand/tile 310. This energy transfer depends on the distance between the fluorophore/quencher on the reporting strand/tile 350 and the fluorophore/quencher on the result indicating strand/tile 310. The status of the target molecule/strand/tile 340 would then be determined based on a comparison of the fluorescence signal with a predetermined range corresponding to a predetermined status of the target molecule/strand/tile 340. This type of multi-level reporting mechanism could thus have application also in other types of molecular systems, such as e.g. systems for molecular biology (e.g. PCR), synthetic biology, and/or DNA/RNA/amino acid nanostructures.

At least one of the reporting strand/tile 350 and the result indicating strand/tile 310 preferably comprises a fluorophore. In embodiments where both the reporting strand/tile 350 and the result indicating strand/tile 310 comprises a fluorophore, the reporting may use FRET.

The described multi-level reporting mechanism may thus be used for many different types of reporting. It may be used for reporting to what extent a specific molecule/strand/tile corresponds to a target molecule/strand/tile, as described above in relation to the determination of to what extent an actual output corresponds to a target output. It may be used for determining the status of a target molecule/strand/tile 340, as described above. As described above, the status of the target molecule/strand/tile 340 may comprise to what extent the result indicating tile/strand 310 binds to the target molecule/strand/tile 340. This may e.g. be used for detecting which of a number of different but closely related molecules/strands/tiles is present in a mixture 100, using the same result indicating strand/tile 310, by designing the result indicating strand/tile 310 to bind to different extents to the different molecules/strands/tiles. The fluorescence signal or level caused by the energy transfer between the reporting strand/tile 350 and the result indicating strand/tile 310 may in this case be used to indicate which one of the different molecules/strands/tiles is present in the mixture 100, by predetermining ranges for the expected fluorescence signal or level for the different molecules/strands/tiles.

In the arrangement 300 in Fig. 3b, the result indicating tile/strand 310 is designed to bind directly to a scaffold position 120 on a scaffold 110, and/or the reporting strand/tile 350 is designed to bind directly to the reporting position 320 on the scaffold 110. This enables a simple detection of whether a specific scaffold 110 is present in a mixture 100. In this embodiment, the determination of the status of the target molecule/strand/tile 340 in the molecular mixture 100 thus comprises determining whether a specific target molecule/strand/tile 340 in the form of a specific scaffold 110 is present in the mixture 100. In the same way as explained above, this may be used for detecting which of a number of different but closely related scaffolds 110 is present in a mixture 100, using the same result indicating strand/tile 310, by designing the result indicating strand/tile 310 to bind in different extents to the scaffold position 120 next to the reporting position 320 on the different scaffolds 110. The fluorescence signal or level caused by the energy transfer between the reporting strand/tile 350 and the result indicating strand/tile 310 may in this case be used to indicate which one of the different scaffolds 110 is present in the mixture 100, by predetermining ranges for the expected fluorescence signal or level for the different scaffolds 110. This may e.g. be used for detecting the presence of a specific virus from a set of closely related viruses in a sample.

The described multi-level reporting mechanism is thus based on the interaction between either a fluorophore and a quencher or two fluorophores, and uses this interaction to determine various aspects of the co-location of two tiles/strands/molecules. As explained above, this may have many different applications.

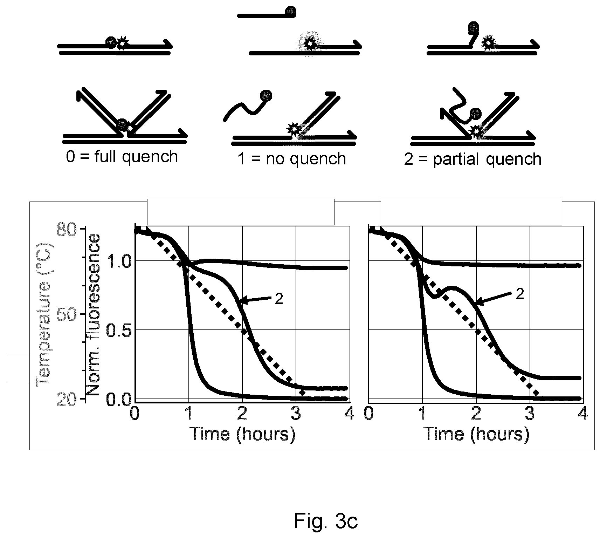

Fig. 3c schematically illustrates temperature curves simplifying the detection of multi-level fluorescence by detecting variations in the fluorescence signal or level at one or more selected temperatures. It is possible to make this detection at only one suitably selected temperature, but preferably the fluorescence signal or level at more than one selected temperatures is detected. Using a typical "heat up, cool down" annealing protocol, program result 2 will occur later during the cool-down-process than program result 0, and this can be detected.

In a similar way, more than three different outcomes can also be detected. The binding strength of the result indicating tile 310 may be varied by varying the length, and/or the sequence, of the result indicating tile 310, in order to programmatically vary the output signal to give signals of different levels. Figs. 3a-c show examples having three levels, but this design can be arbitrarily varied to give an arbitrary number of levels by changing the binding strength of the Q* domain or T* domains.

### Molecular computation examples

A Finite State Machine (FSM) is an important subclass of computer programs, amenable to molecular implementation. Any FSM can be compiled into a one-dimensional SDC. Any 2-state 8-bit input FSM may be implemented by an SDC with *N* = 4, *I* = 3 (meaning 4 scaffold positions 120 for computing and ≤ 3 bits per compute domain 150). For *N* = 4, a total of five binding domains (including the anchor position 125) are typically required on the scaffold 110.

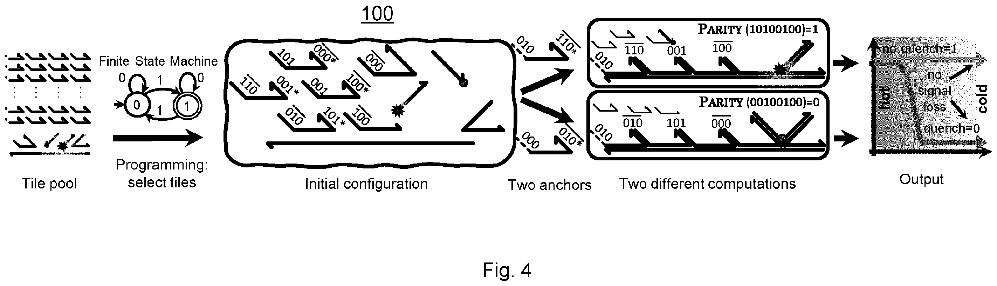

Fig. 4 schematically illustrates the programming of a FSM, using molecular computation and starting with a scaffold 110 and a pool of computing tiles 140. The programming is effected by selecting the computing tiles 140 to use for the computation, from the pool of computing tiles 140. The compute domains 150 of the computing tiles 140 encode both the input and the program, i.e. both data and program bits. Running a program with a different input is effected by changing the set of computing tiles 140, and changing the program is also effected by changing the set of computing tiles 140. The SDC will be reprogrammable.

Two different anchor tiles 130 enables two different PARITY computations, where the target output is shown to the right. The two PARITY examples emphasise that the output in a PARITY computation depends on every bit of input (input 10100100 reports output 1; input 00100100 reports output 0). A PARITY computation may be effected without the use of an anchor tile 130.

A thermodynamically based sequence design approach may be used for compute and reporting domains. However, the scaffold may use biologically-sourced subsequences of M13 bacteriophage chosen to give reasonably isoenergetic position domains.

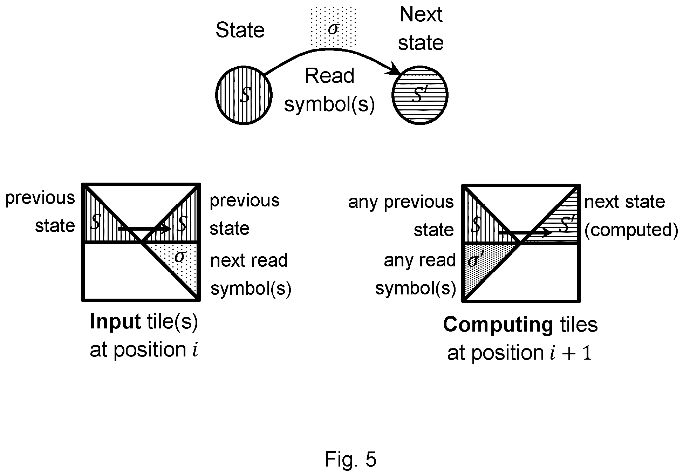

Fig. 5 schematically illustrates a general compilation scheme from a Finite State Machine into computing tiles 140, where each compute domain 150 encodes an FSM state and transition. According to the compilation scheme schematically illustrated in Fig. 5, input bits and program bits are encoded in two separate sets of computing tiles 140, e.g. with input bits at odd scaffold positions and program bits at even scaffold positions. This allows for renewing a program by resetting an input, without requiring the re-supply of program bits. It is however also possible to allow both input and program bits to be encoded in a single compute domain 150.

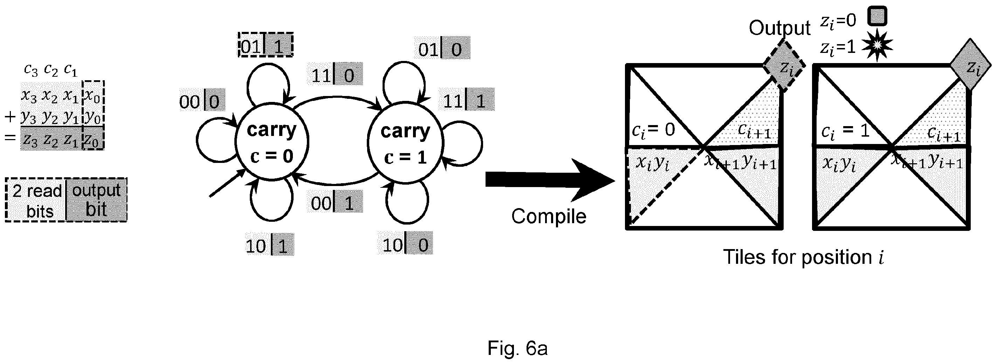

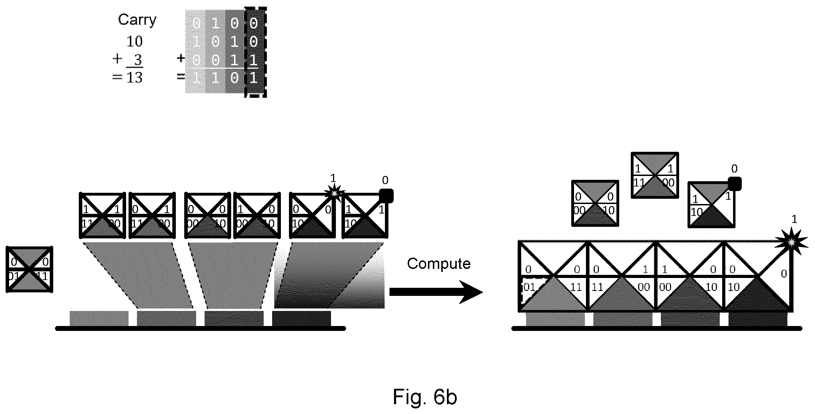

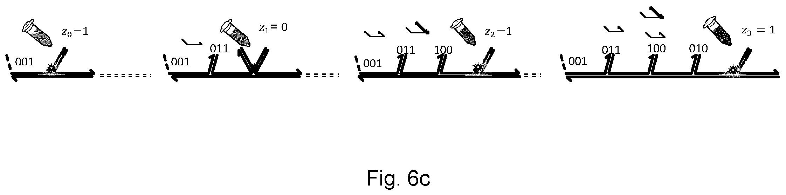

Figs. 6a-c schematically illustrate an example ADDITION program in the form of a 4-bit addition. The addition FSM adds two binary numbers *x* + *y = z*, where each transition reads two bits, *xᵢ* and *yᵢ*, writes output bit *zᵢ*, and enters carry state *cᵢ*. Fig. 6a schematically illustrates how a 4-bit addition FSM is compiled into a 4-bit addition SDC with two computing tiles 140 per scaffold position. In an ADDITION program, each compute domain 150 stores two input bits *xᵢ*, *yᵢ*, and a carry *cᵢ* computed, via choice of computing tile 140, as the mod 2 sum of the previous carry and input pair. In each step, there are two possibilities for the carry, and two possibilities for each outcome. The programming of the SDC is thus effected by translating the FSM into the computing tiles 140 required for effecting the computation. The selected computing tiles are then added to the mixture, and the computation is effected. Fig. 6c schematically illustrates the computation of 10+3=13 in binary using the SDC. The 4 output bits are then read out in four separate experiments, as schematically illustrated in Fig. 6c.

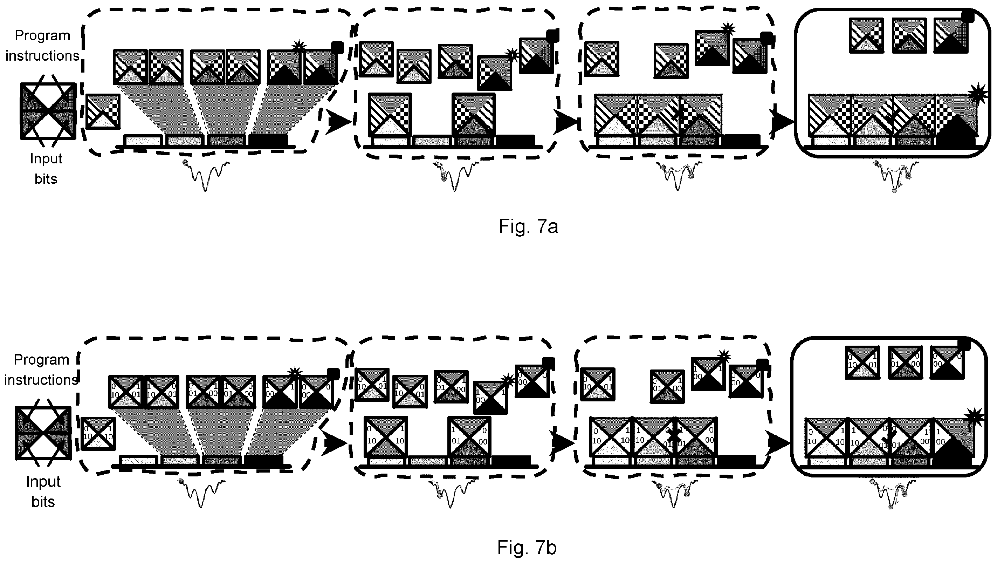

Figs. 7a-b schematically illustrate a molecular computation, illustrated by an example PARITY program that computes whether the number of 1s in an 8-bit input is odd or even. Fig. 7a schematically illustrates the computation using patterns on the compute domains 150, and Fig. 7b schematically illustrates the computation using binary numbers on the compute domains 150.

There are 28 = 256 possible inputs, each corresponding to a different subset of 7 computing tiles 140. Each of four compute domains 150 encodes two input bits, and a variable parity bit computed by correct bindings. A target output for the program is either a single bit at the last position (for e.g. a PARITY program), or an N-bit output along the entire sequence of computing tiles 140 (for e.g. an ADDITION program).

Custom-developed minimum free energy (MFE) and partition function algorithms show the target output having arbitrarily high probability over all other scaffolded configurations. Mathematically, the system scales well: compute domain strength need only be logarithmic in N.

### Experimental results

To test the hypothesis that a thermodynamically favoured computing system can be run using simple annealing protocols, experiments were single-pot and simple, a so-called typical-anneal dropped from 80°C to 20°C in three hours, then held for 45 minutes to obtain a signal completion level. Flat completion levels and reasonably high/low signal suggested a lack of kinetic traps and signal loss (leak). Fast computations may be possible, since particular, slow, kinetic pathways are not enforced. Super-fast anneals, dropping from 80°C to 55°C in under 1 minute, resulted in clear separation of bits 0 and 1. This demonstrates that the SDC can compute as fast as one minute, or even half a minute.

Data was minimally processed: (1) Each raw fluorescence trace was normalised by dividing by its mean (10 datapoints) at 80°C; (2) traces were averaged across at least 2 repeats; and (3) the y-axis was re-scaled so that 0.0 was the mean completion level (at 20 °C) of bit-0-reporting controls, and 1.0 was the mean of bit-1-reporting controls. Control samples had one computing tile 140 per scaffold position 110, hence no per-position competition, and no computation - the ideal target output was simply annealed.

SDC performance, or yield, was estimated on both typical and super-fast anneals using two definitions: (1) the ability to separate bit-0 from bit-1 and, (2) the proportion of correctly assembled target outputs. For (1), typical-anneal data was 100% linearly separable, not only for ADDITION but for all typical-anneal computations executed: all completion levels above 0.47 (fitted on control data) correspond to bit-1, and all below to bit-0. Super-fast anneals are also linearly separable when a threshold is individually fitted for each program. For (2), yield was estimated in several ways which gave similar results, the most straightforward being mean distance between completion levels and target values (0.0 or 1.0): Typical-anneals had mean 96.7% (SD=0.027) yield over all ADDITION inputs (the single worst ADDITION output bit gave 91.6%), with mean 95.3% (SD=0.035) over all computations. Superfast anneals had mean 82.4% (SD=0.116) yield at 1-minute for ADDITION and 81.2% (SD=0.16) over all computations.

Algorithmically, a somewhat liberal approach to kinetics may be taken by allowing each computation to nondeterministically choose from a large set of pathways to the output. Although the SDC can compute isothermally in theory (albeit slowly in practice), in typical anneals rapid kinetics and high yield has been seen, and a lack of subsequent slow completion. A strand design without intentional kinetic traps may be used, but without any design optimisations, or characterisation and prevention of off-pathway interactions (approaches common in typical non-equilibrium molecular computing). The proposed approach is perhaps akin to that in DNA origami, where assembly kinetics is not precisely controlled yet the design works beautifully. Super-fast 1-minute anneals have been run at the speed limit of the laboratory equipment used, and beaten all previous speed records for non-trivial DNA computations. Results for the super-fast, and typical, anneals suggest the SDC has fast, high-temperature, reversible assembly kinetics.

Although the earliest DNA motor was reusable, designing renewable molecular computers is a major challenge due to system complexity, out-of-equilibrium operation, and manual preparation steps. Three SDC programs have been successfully renewed up to 24 times at a reasonable speed of 12 mins per annealing cycle (plus time to add inputs). Even faster cycling is likely possible at the cost of a weaker signal. An acoustic liquid handler may allow for low volume transfers, but manually-pipetted larger volume renewals have been used for several programs with good results. Overall, the principle of thermodynamic favourability provides a simple and effective method for program renewal, beating previous records in: (i) number of cycles on a complex programming platform, (ii) time per renew, (iii) need of extra technology beyond DNA (enzymes, photo/pH regulation), (iv) manual preparation steps.

Thermodynamic computing principles are applicable to nanoscale engineering, for example endowing scaffolded DNA data storage platforms, like SIMD||DNA, with thermodynamic biases against errors. 2D or 3D scaffolded DNA origami could be endowed with computational abilities to drive formation of one of several shapes. Isothermal computation in DNA origami could leverage recent non-computational work.

The SDC gives a method to replace non-equilibrium molecular programs with equilibrium ones for the same task. But there are limitations: fuel-consuming, infinite-time, out-of- equilibrium molecular dynamics are, by definition, unsuited to thermodynamically favoured computation. However, even there, energy landscapes can be sculpted to encode complex, finite-time, dynamics while driving to a ground state - for example by running a fixed-count chemical oscillator.

Inherent robustness to temperature and concentration, and lack of leak/errors, could facilitate thermodynamic computing in complex biological environments, engineered in RNA or protein. Indeed, a biologically-sourced scaffold has been used as a proof-of-principle for under-designed DNA sequences. Beyond molecular programming, the theory of thermodynamics of computation seeks to determine ultimate energetic costs for computation. The SDC has several costs (number of synthesised strands, strand length, heat/annealing, etc.), but does not require explicit error-correction scale-up/time/energy/material costs, which seem to dominate computing architectures be they molecular, classical digital-electronic or quantum.

### Method embodiments

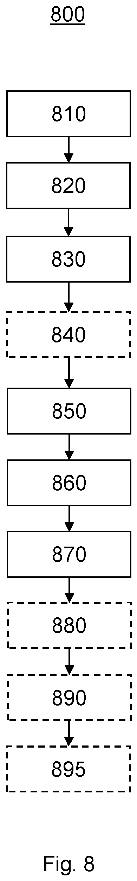

Fig. 8 schematically illustrates a method 800 for making molecular computations. The method 800 may comprise:
Step 810: providing a mixture 100 comprising a scaffold 110, comprised of a long information-encoding molecule or strand, such as e.g. a long polynucleotide strand or a long amino acid sequence, and comprising N binding domains, wherein each binding domain constitutes a scaffold position 120 required for the computation.
Step 820: designing a set of computing tiles to drive a desired computation, the set of computing tiles comprising at least N different computing tile types, wherein the computing tile types are selected to be used for the computation, in such a way that a target output has a higher probability of being reached than the probability for any other potential output, wherein each computing tile 140 is comprised of a short information-encoding molecule or strand, such as e.g. a short polynucleotide or a short amino acid sequence, and comprises a bottom position domain 160 and at least one compute domain 150, wherein for each computing tile 140, the bottom position domain 160 is arranged to bind directly to a matching scaffold position 120 on the scaffold 110 with a first binding strength, and to the compute domains 150 of other computing tiles 140 with a set of second binding strengths, which are each weaker than the first binding strength. The target output may even have a higher probability of being reached than the sum of the probabilities for all other potential outputs.
Step 830: adding the designed set of computing tiles to the mixture 100.
Step 850: allowing bottom position domains 160 of computing tiles 140 to bind to scaffold positions 120 until all scaffold positions 120 required for the computation have been filled.
Step 860: allowing replacement of all mismatched computing tiles 140, based on correct compute domain bindings being enthalpically favoured over incorrect compute domain bindings.
Step 870: reaching an output configuration.

This avoids the need for extra error-correction subsystems, since the molecular computing naturally evolves to the target output configuration at equilibrium.

In embodiments, the designing 820 comprises ensuring that there for each computing tile type is a selected concentration of computing tiles 140 in the mixture 100, the selected concentration ensuring that there is an excess of computing tiles 140 of the computing tile type in the mixture 100. This ensures that there will always be a suitable computing tile 140 of the correct computing tile type available to bind to the scaffold position 120, and also makes the method 800 less vulnerable to impurities in the computing tiles 140. All computing tiles 140 of the same computing tile type are preferably substantially identical.

In embodiments, the providing 810 comprises arranging the binding domains on the scaffold to be unique. However, many or all of the binding domains on the scaffold may alternatively be similar to each other.

In embodiments, the providing 810 comprises arranging the binding domains on the scaffold to all have approximately the same length and/or binding strength. However, the binding domains are not necessarily fixed domains on the scaffold. They may even in embodiments have a variable length and/or binding strength.

In embodiments, the scaffold 110 further comprises an additional binding domain in the form of an anchor position 125, and the set of computing tiles further comprises an anchor tile 130 comprising a bottom position domain 180 and an anchoring compute domain 170. The allowing 850 may then further comprise allowing the bottom position domain 180 of the anchor tile 130 to bind to the anchor position 125. The anchor tile 130 does not have to be added at the beginning of the computation, it is possible to add it at a later stage (especially in embodiments where the mixture is not heated), but this will make the computation slower.

In embodiments, the designing 820 comprises designing the second binding strengths in the set of second binding strengths to be approximately equal to each other.

The method 800 may further comprise one or more of:
Step 840: heating the mixture 100 to release all bindings. The allowing 850 of bottom position domains 160 of computing tiles 140 to bind to scaffold positions 120, and the allowing 860 of replacement of all mismatched computing tiles 140, then preferably take place as the mixture 100 cools. The reaching 870 of an output configuration will in this case typically take place when the mixture has cooled. This allows for a much quicker computation. It is however not necessary to heat and cool the mixture, it is perfectly possible to let the mixture instead have a constant temperature. In this case, a suitable temperature should be selected (the temperature may e.g not be so high that the bindings are released).
Step 880: reporting the outcome of the molecular computation by reporting the detected fluorescence signal or level caused by the energy transfer between the reporting tile 350 and the result indicating tile 310, when the reporting tile 350, directly or via the intermediate tile 330, has bound to the reporting position 320, and the result indicating tile 310 has bound to the actual final compute domain 150 (if the providing 810 further comprises providing a reporting tile 350, preferably comprising a fluorophore or a quencher, i.e. having an attached fluorophore/quencher "label", in the mixture, wherein the reporting tile 350, directly or via an intermediate tile 330, is arranged to bind to a reporting position 320, which is a further scaffold position immediately following the final scaffold position 120 required for the computation; the designing 820 further comprises designing a result indicating tile 310, preferably comprising a fluorophore or a quencher, i.e. having an attached fluorophore/quencher "label", to bind to the target final compute domain 150; and the adding 830 further comprises adding the result indicating tile 310 to the mixture).

At least one of the reporting tile 350 and the result indicating tile 310 preferably comprises a fluorophore. In embodiments where both the reporting tile 350 and the result indicating tile 310 comprises a fluorophore, the reporting may use FRET.

The detection of more than two different outcomes may comprise detecting variations in the fluorescence signal or level at one or more selected temperatures.

Step 890: allowing further computations by resetting the mixture 100 by adding at least one blocking computing tile to the mixture 100, wherein each added blocking computing tile is arranged to bind to the bottom position domain 180 and to at least one compute domain 150, 170 of a selected computing tile 130, 140, thereby blocking the selected computing tile 130, 140 from being used in the computation. The mixture 100 may be heated to release all bindings before adding the at least one blocking computing tile, but this is not necessary - it is perfectly possible to let the mixture 100 instead have a constant temperature, even during resetting of the mixture 100.

Step 895: repeating steps ii-iv.

The above steps may be effected in any order that makes technical sense. For example, the scaffold does not have to be present in the mixture before the set of computing tiles is added. Some of the steps may also be effected simultaneously with each other.

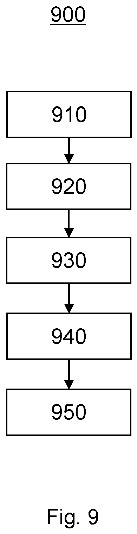

Fig. 9 schematically illustrates a method 900 for determining the status of a target molecule 340 in a molecular mixture 100. The method 900 may comprise:
Step 910: adding a reporting strand 350, preferably comprising a fluorophore or a quencher, i.e. having an attached fluorophore/quencher "label", which may bind to an intermediate strand 330, to a molecular mixture 100.
Step 920: designing a result indicating strand 310, preferably comprising a fluorophore or a quencher, i.e. having an attached fluorophore/quencher "label", to bind to a predetermined extent to a target molecule 340.
Step 930: adding the result indicating strand 310 to the molecular mixture 100.
Step 940: detecting the fluorescence signal or level caused by the energy transfer between the reporting strand 350 and the result indicating strand 310. This energy transfer depends on the distance between the fluorophore/quencher on the reporting strand 350 and the fluorophore/quencher on the result indicating strand 310. At least one of the reporting strand 350 and the result indicating strand 310 preferably comprises a fluorophore. In embodiments where both the reporting strand 350 and the result indicating strand 310 comprises a fluorophore, the reporting may use FRET.
Step 950: determining the status of the target molecule 340 based on a comparison of the fluorescence signal with a predetermined range corresponding to a predetermined status of the target molecule 340.

In embodiments, the status of the target molecule 340 comprises to what extent the result indicating strand 310 binds to the target molecule.

In embodiments, the target molecule 340 is a part of a predetermined set of different molecules, the result indicating strand 310 is arranged to bind to different extents to each molecule in the predetermined set of different molecules, and the status of the target molecule 340 comprises the presence of the target molecule 340 in the molecular mixture 100.

In embodiments, the target molecule 340 is a scaffold 110, the reporting strand 350 is designed to bind to a reporting position 320 on the scaffold 110, and the result indicating strand 310 is designed to bind to a predetermined extent to a scaffold position 120 close to the reporting position 320.

In embodiments, the reporting strand 350 binds to an intermediate strand 330, which is designed to bind to the target molecule 340.

At least one of the reporting strand 350 and the result indicating strand 310 preferably comprises a fluorophore. In embodiments where both the reporting strand 350 and the result indicating strand 310 comprises a fluorophore, the reporting may use FRET

In embodiments, the target molecule 340 comprises a target final compute domain 150 of a computing tile 140.

Detection of more than two different outcomes may comprise detecting variations in the fluorescence signal or level at one or more selected temperatures.

The foregoing disclosure is not intended to limit the present invention to the precise forms or particular fields of use disclosed. It is contemplated that various alternate embodiments and/or modifications to the present invention, whether explicitly described or implied herein, are possible in light of the disclosure. The molecular computer has been described in the form of a DNA computer, but any other kind of information-encoding molecules or strands, such as e.g. polynucleotide strands or amino acid sequences, such as e.g. in the form of RNA or protein, may alternatively be used. The number N of scaffold positions is for illustrative purposes rather low in the illustrated embodiments, but will for practical purposes typically be much larger. Embodiments with N=20, N=100, N=500, N=1000, N>1000, or any integer in between are envisioned. Accordingly, the scope of the invention is defined only by the claims.

## Claims

1. A method (800) for making molecular computations, the method (800) comprising:
i. providing (810) a mixture (100) comprising a scaffold (110), comprised of a long information-encoding molecule or strand, such as e.g. a long polynucleotide strand or a long amino acid sequence, and comprising N binding domains, wherein each binding domain constitutes a scaffold position (120);
ii. designing (820) a set of computing tiles to drive a desired computation, the set of computing tiles comprising at least N different computing tile types, wherein the computing tile types are selected to be used for the computation, in such a way that a target output has a higher probability of being reached than the probability for any other potential output, where each computing tile (140) is comprised of a short information-encoding molecule or strand, such as e.g. a short polynucleotide or a short amino acid sequence, and comprises a bottom position domain (160) and at least one compute domain (150), wherein for each computing tile (140), the bottom position domain (160) is arranged to bind directly to a matching scaffold position (120) on the scaffold (110) with a first binding strength, and to the compute domains (150) of other computing tiles (140) with a set of second binding strengths, which are each weaker than the first binding strength;
iii. adding (830) the set of computing tiles to the mixture (100);
iv. allowing (850) bottom position domains (160) of computing tiles (140) to bind to scaffold positions (120), until all scaffold positions (120) required for the computation have been filled;
v. allowing (860) replacement of all mismatched computing tiles (140), based on correct compute domain bindings being enthalpically favoured over incorrect compute domain bindings; and
vi. reaching (870) an output configuration.

2. A method (800) according to claim 1, further comprising heating (840) the mixture (100) to release all bindings, wherein the allowing (850) of bottom position domains (160) of computing tiles (140) to bind to scaffold positions (120), and the allowing (860) of replacement of all mismatched computing tiles (140), take place as the mixture (100) cools, and the reaching (870) of the output configuration takes place when the mixture has cooled.

3. A method (800) according to any one of claims 1-2, wherein:
(i) wherein the designing (820) comprises ensuring that there for each computing tile type is a selected concentration of computing tiles (140) in the mixture (100), the selected concentration ensuring that there is an excess of computing tiles (140) of the computing tile type in the mixture (100).
(ii) the providing (810) comprises arranging the binding domains on the scaffold to be unique;
(iii) the providing (810) comprises arranging the binding domains on the scaffold to all have approximately the same length and/or binding strength;
(iv) the scaffold (110) further comprises an additional binding domain in the form of an anchor position (125), and the set of computing tiles further comprises an anchor tile (130) comprising a bottom position domain (180) and an anchoring compute domain (170), wherein the allowing (850) further comprises allowing the bottom position domain (180) of the anchor tile (130) to bind to the anchor position (125); and/or
(v) wherein the designing (820) comprises designing the second binding strengths in the set of second binding strengths to be approximately equal to each other.

4. A method (800) according to any one of claims 1-3, further comprising allowing further computations by:
vii. resetting (890) the mixture (100) by adding at least one blocking computing tile to the mixture (100), wherein each added blocking computing tile is arranged to bind to the bottom position domain (160, 180) and to at least one compute domain (150, 170) of a selected computing tile (130, 140), thereby blocking the selected computing tile (130, 140) from being used in the computation; and
viii. repeating (895) steps ii-vi.

5. A method (800) according to any one of claims 1-4, wherein:
the providing (810) further comprises providing a reporting tile (350) in the mixture, wherein the reporting tile (350), directly or via an intermediate tile (330), is arranged to bind to a reporting position (320), which is a further scaffold position immediately following the final scaffold position (120) required for the computation;
the designing (820) further comprises designing a result indicating tile (310) to bind to the target final compute domain (150); and
the adding (830) further comprises adding the result indicating tile (310) to the mixture,
the method further comprising reporting (880) the outcome of the molecular computation by reporting the detected fluorescence signal caused by the energy transfer between the reporting tile (350) and the result indicating tile (310), when the reporting tile (350), directly or via the intermediate tile (330), has bound to the reporting position (320), and the result indicating tile (310) has bound to the actual final compute domain (150); and optionally or preferably,
wherein the reporting tile (350) and/or the result indicating tile (310) comprises a fluorophore; and/or
wherein detection of more than two different outcomes comprises detecting variations in the fluorescence signal at one or more selected temperatures.

6. Arrangement (200) for making molecular computations, the arrangement (200) comprising:
a mixture (100) comprising a scaffold (110), comprised of a long information-encoding molecule or strand, such as e.g. a long polynucleotide strand or a long amino acid sequence, and comprising N binding domains, each binding domain constituting a scaffold position (120); and
a set of computing tiles, which has been designed to drive a desired computation, wherein the designed set of computing tiles comprises at least N different computing tile types, which have been selected to be used for the computation in such a way that a target output has a higher probability of being reached than the probability for any other potential output, each computing tile (140) comprised of a short information-encoding molecule or strand, such as e.g. a short polynucleotide or a short amino acid sequence, and comprising a bottom position domain (160) and at least one compute domain (150), wherein for each computing tile (140), the bottom position domain (160) is arranged to bind directly to a matching scaffold position (120) on the scaffold (110) with a first binding strength, and to the compute domains (150) of other computing tiles (140) with a set of second binding strengths, which are each weaker than the first binding strength;
wherein the arrangement (200) is configured to:
allow bottom position domains (160) of computing tiles (140) to bind to scaffold positions (120) until all scaffold positions (120) required for the computation have been filled; and
allow replacement of all mismatched computing tiles (140), based on correct compute domain bindings being arranged to be enthalpically favoured over incorrect compute domain bindings, thereby allowing an output configuration to be reached.

7. An arrangement (200) according to claim 6, wherein:
(i) there for each computing tile type is a selected concentration of computing tiles (140) in the mixture (100), the selected concentration ensuring that there is an excess of computing tiles (140) of the computing tile type in the mixture (100);
(ii) the arrangement (200) is further configured to heat the mixture (100) to release all bindings, wherein the allowing of bottom position domains (160) of computing tiles (140) to bind to scaffold positions (120), and the allowing of replacement of all mismatched computing tiles (140), take place as the mixture (100) cools, and the reaching of the output configuration takes place when the mixture has cooled;
(iii) the binding domains on the scaffold are unique;
(iv) the binding domains on the scaffold all have approximately the same length and/or binding strength;
(v) the scaffold (110) further comprises an additional binding domain in the form of an anchor position (125), and the set of computing tiles further comprises an anchor tile (130) comprising a bottom position domain (180) and an anchoring compute domain (170), and the bottom position domain (180) of the anchor tile (130) is configured to bind to the anchor position (125);
(vi) the second binding strengths in the set of second binding strengths are approximately equal to each other; and/or
(vii) wherein the arrangement (200) is configured to allow further computations by:
resetting the mixture (100) by at least one blocking computing tile being added to the mixture (100), wherein each added blocking computing tile is arranged to bind to the bottom position domain (160, 180) and to at least one compute domain (150, 170) of a selected computing tile (130, 140), thereby blocking the selected computing tile (130, 140) from being used in the computation; and
allowing one or more new computing tiles (130, 140) to be added to the mixture (100).

8. An arrangement (200) according to any one of claims 6-7, wherein the mixture (100) further comprises:
a reporting tile (350), which is designed to, directly or via an intermediate tile (330), bind to a reporting position (320), which is a further scaffold position immediately following the final scaffold position (120) required for the computation; and
a result indicating tile (310), designed to bind to the target final compute domain (150),
wherein the arrangement (200) is configured to report the outcome of the molecular computation by reporting the detected fluorescence signal caused by the energy transfer between the reporting tile (350) and the result indicating tile (310), when the reporting tile (350), directly or via the intermediate tile (330), has bound to the reporting position (320), and the result indicating tile (310) has bound to the actual final compute domain (150).

9. An arrangement (200) according to claim 8, wherein:
(i) the reporting tile (350) and/or the result indicating tile (310) comprises a fluorophore; and/or
(ii) detection of more than two different outcomes comprises detecting variations in the fluorescence signal at one or more selected temperatures.

10. Method (900) for determining the status of a target molecule (340) in a molecular mixture (100), the method comprising:
adding (910) a reporting strand (350) to a molecular mixture (100);
designing (920) a result indicating strand (310) to bind to a predetermined extent to a target molecule (340);
adding (930) the result indicating strand (310) to the molecular mixture (100);
detecting (940) the fluorescence signal caused by the energy transfer between the reporting strand (350) and the result indicating strand (310); and
determining (950) the status of the target molecule (340) based on a comparison of the fluorescence signal with a predetermined range corresponding to a predetermined status of the target molecule (340).

11. Method (900) according to claim 10, wherein:
(i):
the target molecule (340) is a part of a predetermined set of different molecules;
the result indicating strand (310) is arranged to bind to different extents to each molecule in the predetermined set of different molecules; and
the status of the target molecule (340) comprises the presence of the target molecule (340) in the molecular mixture (100); and/or
(ii):
the target molecule (340) is a scaffold (110);
the reporting strand (350) is designed to bind to a reporting position (320) on the scaffold (110); and
the result indicating strand (310) is designed to bind to a predetermined extent to a scaffold position (120) close to the reporting position (320).

12. Method (900) according to any one of claims 10-11, wherein:
(i) wherein the status of the target molecule (340) comprises to what extent the result indicating strand (310) binds to the target molecule (340);
(ii) the reporting strand (350) binds to an intermediate strand (330), which binds to a reporting position (320) on a scaffold (110);
(iii) the reporting strand (350) and/or the result indicating strand (310) comprises a fluorophore;
(iv) the target molecule (340) comprises a target final compute domain (150) of a computing tile (140); and/or
(v) the detection of more than two different outcomes comprises detecting variations in the fluorescence signal at one or more selected temperatures.

13. Arrangement (300) for determining the status of a target molecule (340) in a molecular mixture (100), the arrangement (300) comprising a molecular mixture (100) comprising a reporting strand (350) and a result indicating strand (310), which has been designed to bind to a predetermined extent to the target molecule (340), wherein the arrangement (300) is configured to detect the fluorescence signal caused by the energy transfer between the reporting strand (350) and the result indicating strand (310), and determine the status of the target molecule (340) based on a comparison of the fluorescence signal with a predetermined range corresponding to a predetermined status of the target molecule (340).

14. Arrangement (300) according to claim 13, wherein:
(i):
the target molecule (340) is a part of a predetermined set of different molecules;
the result indicating strand (310) is arranged to bind to different extents to each molecule in the predetermined set of different molecules; and
the status of the target molecule (340) comprises the presence of the target molecule (340) in the molecular mixture (100); and/or
(ii):
the target molecule (340) is a scaffold (110);
the reporting strand (350) is designed to bind to a reporting position (320) on the scaffold (110); and
the result indicating strand (310) is designed to bind to a predetermined extent to a scaffold position (120) close to the reporting position (320).

15. Arrangement (300) according to any one of claims 13 or 14, wherein:
(i) the status of the target molecule (340) comprises to what extent the result indicating strand (310) binds to the target molecule (340);
(ii) the reporting strand (350) binds to an intermediate strand (330), which is designed to bind to the target molecule (340);
(iii) the reporting strand (350) and/or the result indicating strand (310) comprises a fluorophore;
(iv) the target molecule (340) comprises a target final compute domain (150) of a computing tile (140); and/or
(v) the detection of more than two different outcomes comprises detecting variations in the fluorescence signal at one or more selected temperatures.
